# FASCICULE DE BREVET EUROPEEN

(11) **EP 1 064 930 B1**
(45) Date de publication et mention de la délivrance du brevet: **06.02.2002**
(21) Numéro de dépôt: 00401619.2
(22) Date de dépôt: 08.06.2000
(51) Int. Cl.: A61K 7/48, A61K 7/02

(54) **Composition de soin ou de maquillage contenant des fibres et un organopolysiloxane hydrophile**
Fasern und ein hydrophiles Organopolysiloxan enthaltende Pflege- oder Schminkzusammensetzung
Care or make-up composition containing fibers and a hydrophilic organopolysiloxane

(30) Priorité: 01.07.1999 FR 9908489
(43) Date de publication de la demande: 03.01.2001
(73) Titulaire: L'OREAL, 75008 Paris (FR)
(72) Inventeur: Jager-Lezer, Nathalie, 92340 Bour-la-Reine (FR)
(74) Mandataire: Lhoste, Catherine

(56) Documents cités:
- EP-A- 0 293 795
- EP-A- 0 295 886

## Description

La présente invention se rapporte à une composition contenant des fibres et une dispersion aqueuse d'un organopolysiloxane, destinée aux domaines cosmétique et dermatologique. Plus spécialement, l'invention s'applique au soin et/ou au traitement et/ou maquillage des matières kératiniques comme la peau, y compris le cuir chevelu, les lèvres du visage et les phanères comme les cils, les sourcils, les ongles et les cheveux des êtres humains. Cette composition est douce et fraîche à l'application, s'étale facilement, est non-collante et n'est pas desséchante pour la peau et les lèvres. Elle est parfaitement adaptée aux peaux grasses, du fait de son haut pouvoir matifiant.

Cette composition peut se présenter notamment sous forme de produit coulé en stick ou en coupelle comme les rouges ou baumes à lèvres, les fonds de teint coulés, les produits anticerne, les déodorants, les fards à paupières ou à joues ; sous forme de pâte ou de crème plus ou moins fluide comme les fonds de teint ou rouges à lèvres fluides, les eye-liners, les mascaras, les compositions de soin, de protection solaire ou de coloration de la peau, de maquillage du corps ou encore de maquillage des ongles et des cheveux ; sous forme de gel aqueux comme les shampooings traitant.

Il est connu d'utiliser des fibres dans des produits de maquillage notamment pour leurs effets allongeant dans des mascaras (voir JP-A-57/158714), leur toucher « textile» (voir JP-A-7/196440), leur effet de tissus ou encore leurs propriétés hydratantes dans des rouges à lèvres (voir le document US-A-5 498 407) ou pour améliorer les contours du rouge à lèvres sur les bords des lèvres (voir le document EP-A-0 106 762). Malheureusement, il est très difficile de disperser des fibres dans des compositions, de façon homogène et sans former d'amas, ce qui dans une composition colorée et en particulier de maquillage confère généralement un maquillage non uniforme et peu esthétique, à contour peu net. En outre, cette difficulté de dispersion conduit à des compositions de propriétés cosmétiques non constantes et peu reproductibles, ce qui entraîne des problèmes de fabrication industrielle et des coûts élevés de fabrication.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques ou dermatologiques. Les charges servent généralement à modifier la texture de la composition et en particulier à la rigidifier ainsi qu'à matifier le film de composition déposé sur la peau et/ou les lèvres, ce qui est particulièrement recherché pour les utilisateurs à peaux mixtes ou grasses, ainsi que sous les climats chauds et humides. En revanche, les pigments servent généralement à apporter de la couleur à la composition. Malheureusement, les charges utilisées pour apporter de la matité n'apportent pas de fraîcheur mais plutôt de la sécheresse à la peau ainsi que des tiraillements et de l'inconfort.

Il subsiste donc le besoin d'une composition ne présentant pas les inconvénients ci-dessus, et notamment ne desséchant pas la peau ou les lèvres sur lesquelles elle est appliquée, aussi bien lors de l'application qu'au cours du temps et conférant un maquillage ou un soin homogène esthétique, un apport de fraîcheur tout en apportant de la matité.

L'invention a justement pour objet une composition de soin et/ou de traitement et/ou de maquillage des matières kératiniques permettant de remédier à ces inconvénients. De façon surprenante, le demandeur a trouvé que l'utilisation de fibres et d'une dispersion aqueuse d'organopolysiloxane en partie réticulé dans une composition de maquillage conférait à la composition de bonnes propriétés cosmétiques, et permettait l'obtention d'un maquillage homogène, uniforme, à contour net, lors de l'application accompagnée d'un toucher doux et d'un effet fraîcheur. Ce maquillage est en outre confortable à porter tout au long de la journée et présente des propriétés matifiantes et de résistance à l'eau.

L'invention s'applique non seulement aux produits de maquillage de la peau, aussi bien du visage que du corps humain, et des lèvres du visage, mais aussi aux produits de maquillage des phanères comme les cils, sourcils, ongles et cheveux ainsi qu'aux produits de soin et/ou de traitement de la peau, y compris du cuir chevelu.

De façon plus précise, l'invention a pour objet une composition de soin ou de maquillage des matières kératiniques, contenant une phase aqueuse, des particules d'organopolysiloxane solide élastomérique au moins partiellement réticulé et des fibres dispersées dans la phase aqueuse.

En particulier, les particules d'organopolysiloxane et les fibres sont dispersées voire solubilisées directement dans la phase aqueuse.

L'invention a encore pour objet un procédé cosmétique de soin ou de traitement des matières kératiniques et notamment de la peau ou des lèvres des êtres humains, comprenant l'application sur ces matières kératiniques de la composition en particulier cosmétique telle que définie ci-dessus.

L'invention a encore pour objet l'utilisation cosmétique de particules d'organopolysiloxane solide élastomérique au moins partiellement réticulé en suspension dans une phase aqueuse, associées à des fibres, dans une composition cosmétique notamment de soin ou de maquillage de matières kératiniques ou pour la fabrication d'une composition à application topique et plus spécialement d'une composition de maquillage ou de soin des matières kératiniques, résistante à l'eau et/ou conférant un maquillage ou un soin homogène et/ou frais et/ou matifiant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500 et mieux de 5 à 150.

Avantageusement, la phase aqueuse contient de l'eau et éventuellement un ou plusieurs composés miscibles au moins en partie à l'eau comme les polyols, les mono alcools inférieurs en C₂ à C₅ et les cétones en C₃ à C₄ liquides à température ambiante.

Par "température ambiante", il faut comprendre une température de 25°C, à pression atmosphérique normale (76mm de Hg).

Par "polyol", il faut comprendre toute molécule organique comportant au moins deux groupements hydroxyle libres. En particulier, le ou les polyols de l'invention présentent une valeur d'IOB (Balance/Inorganique/Organique) supérieure à 0,5 et en particulier allant de 1 à 7 et plus spécialement de 1,5 à 5,5. Ces polyols servent notamment d'agents mouillants des fibres.

Le paramètre IOB est connu de l'homme du métier à partir d'un certain nombre de publications comme l'article de A. FUJITA Pharm. Bull 2, 163-173 (1954) et les documents JO 9/151109, J08/217639 de Shiseido ou J09/175925 de Kose.

A titre d'exemples de polyols qui vérifient les critères précédents, et qui peuvent être utilisés seuls ou en mélange dans la composition de l'invention, on peut citer :

| **Nom** | **Valeur d'IOB** |
|---|---|
| - Propylène glycol | 3,333 |
| - Butylène glycol | 2,500 |
| - Isoprène glycol | 2,222 |
| - Pentylène glycol | 2,000 |
| - Hexylène glycol | 1,818 |
| - PEG-4 (*) | 2,656 |
| - PEG-6 | 2,396 |
| - PEG-8 | 2,266 |
| - Glycérol | 5,000 |
| - Panthénol | 3,125 |

| | |
|---|---|
| (*) De façon générale, on peut citer les polyéthylène glycols (PEG) ayant de 4 à 8 motifs d'éthylène glycol. | |

La phase aqueuse peut représenter de 1 à 98 % du poids total de la composition et de préférence de 10 à 95 et mieux de 30 à 95 %.

Le ou les composés miscibles à l'eau peuvent être présents en une quantité allant de 0 à 30 % du poids total de la composition notamment de 0,1 à 30 % et mieux en une quantité allant de 1 à 15 %.

Cette composition est notamment non collante au toucher, non grasse et douce à l'application, s'étalant bien, tout en étant d'un aspect homogène.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 0,1 mm à 5 mm et mieux de 0,2 mm à 1,6 mm. Leur section peut être comprise dans un cercle de diamètre allant de 2 nm à 100µm, de préférence allant de 20 nm à 50 µm et mieux de 500nm à 50 µm. Le poids ou titre des fibres est souvent donné en denier ou décitex et représente le poids en gramme de 9km de fil. De préférence, les fibres selon l'invention ont un titre choisi dans la gamme allant de 0,15 à 30 deniers et mieux de 0,18 à 18 deniers.

Pour obtenir un maquillage brillant, ce qui tout particulièrement recherché pour le maquillage des ongles et des lèvres, on utilise avantageusement des fibres courtes ayant en particulier une longueur allant de 1 nm à 200µm. En revanche, pour un maquillage mat, ce qui surtout recherché pour le maquillage du visage (notamment pour une poudre ou un fond de teint), on utilise de préférence des fibres longues, ayant notamment une longueur supérieure à 200µm.

Les fibres peuvent être celles utilisées dans la fabrication des textiles et notamment des fibres dé soie, de coton, de laine, de lin, des fibres de cellulose extraites notamment du bois, des légumes ou des algues, de polyamide (Nylon®), de rayonne, de viscose, d'acétate notamment d'acétate de rayonne, de cellulose ou de soie, de poly-p-phénylène téréphtamide notamment de Kevlar®, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de polytétrafluoroéthylène (Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et ε-caprolactone (Monocryl de chez Johnson & Johnson) ; les fibres synthétiques résorbables du type copolymère d'acide lactique et d'acide glycolique (Vicryl de chez Johnson & Johnson) ; les fibres de polyester téréphtalique (Ethibond de chez Johnson & Johnson) et les fils d'acier inoxydable (Acier de chez Johnson & Johnson) notamment pour une application en vernis à ongles.

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet anti-statique (par exemple le R-STAT de chez Rhodia) ou un autre polymère permettant une organisation particulière des fibres (traitement de surface spécifique) ou traitement de surface induisant des effets de couleurs/hologrammes (fibre Lurex de chez Sildorex, par exemple).

De préférence, on utilise des fibres d'origine synthétiques et en particulier des fibres organiques, comme celles utilisées en chirurgie.

Les fibres utilisables dans la composition selon l'invention sont préférentiellement des fibres de polyamide ou de poly-p-phénylène téréphtamide. Leur longueur L peut aller de 0,1 à 5 mm, de préférence de 0,25 à 1,6 mm et leur diamètre moyen D peut aller de 5 à 50 µm. En particulier, on peut utiliser les fibres de polyamide commercialisées par les Etablissements P. Bonte sous le nom Polyamide 0.9 Dtex 0,3 mm, ayant un diamètre moyen de 6 µm, de 12,2 µm ou de 20 µm, un poids d'environ (0,9 dtex) et une longueur allant de 0,3 mm à 1, mm. On peut aussi utiliser les fibres de poly-p-phénylène téréphtamide de diamètre moyen de 12 µm et de longueur d'environ 1,5 mm comme celles vendues sous le nom de Kevlar Floc par la société Du Pont Fibres.

La concentration en fibres est fonction de l'application spécifique et du type de produit envisagé. Pour un produit de maquillage du visage du type fond de teint ou des lèvres (du type rouge à lèvres) la concentration en fibres peut aller de 0,1 à 20% du poids total de la composition, de préférence de 0,5 à 10%. Pour un effet spécial notamment de maquillage du corps, des ongles ou des cheveux, la quantité de fibres peut aller jusqu'à 30% du poids total de la composition.

La composition de l'invention peut se présenter sous forme de pâte, de solide, de crème plus ou moins fluide voire même de lotion. Elle peut être un gel aqueux ou hydrophile, une dispersion ou émulsion huile-dans-eau ou eau-dans-huile fluide, rigide ou souple, éventuellement coulé en stick ou en coupelle.

Par organopolysiloxane « élastomérique » on entend un organopolysiloxane souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymères de haut poids moléculaire dont la mobilité est limitée par un réseau uniforme de points de réticulation.

Les organopolysiloxanes élastomériques de la composition de l'invention présentent des propriétés de structurant de milieu aqueux et sont aptes à augmenter la viscosité de ce milieu aqueux, en plus de bonnes propriétés cosmétiques, notamment de douceur, de fraîcheur et de matité. Ils ne sont pas desséchants pour la peau. Ces nouveaux élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux aqueux et en particulier ceux de la composition et ceux dus à la transpiration de la peau. Ils permettent, en association avec les fibres, l'obtention de compositions plus ou moins épaissies ayant une bonne rémanence à l'eau et une bonne stabilité.

Les organopolysiloxanes élastomériques conformes à l'invention sont des composés hydrophiles partiellement ou totalement réticulés et de structure tridimensionnelle. L'épaississement de la phase aqueuse par ces élastomères peut être totale ou partielle. Il est tout à fait surprenant que des polymères hydrophiles associés à des fibres présentent des propriétés de rémanence à l'eau.

Les élastomères de la composition de l'invention se présentent sous forme de poudre ou de gel émulsionné contenant un organopolysiloxane élastomère de structure tridimensionnelle, dispersé dans de l'eau. La dispersion (ou suspension) des particules est homogène.

Les organopolysiloxanes élastomériques selon l'invention peuvent être choisis parmi les polymères réticulés décrits dans la demande JP-A-10/175816. Selon cette demande, ils sont obtenus par réaction d'addition et de réticulation, en présence d'un catalyseur notamment du type platine, d'au moins :
- (a) un organopolysiloxane (i) ayant au moins deux groupes vinyliques en position α-ω de la chaîne siliconée par molécule ; et
- (b) un organosiloxane (ii) ayant au moins un atome d'hydrogène lié à un atome de silicium par molécule.

En particulier, l'organopolysiloxane (i) est choisi parmi les polydiméthylsiloxanes et est plus spécifiquement un α-ω-diméthylvinyl polydiméthylsiloxane.

Les organopolysiloxanes élastomériques de la composition selon l'invention se présente avantageusement sous forme de suspension ou dispersion aqueuse. Cette suspension peut être notamment obtenue comme suit
- (a) mélange de l'organopolysiloxane (i) et de l'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

L'eau est avantageusement ajoutée à une température de 40-60°C. Après l'étape (e), il est possible de sécher les particules obtenues, pour en évaporer toute ou partie de l'eau piégée.

Les organopolysiloxanes sont sous la forme de particules solides déformables hydrophiles ayant une certaine dureté, mesurable avec un duromètre Shore A (selon la norme ASTM D2240) à la température ambiante ou avec la méthode japonaise JIS-A. Cette dureté peut être mesurée sur un bloc d'élastomère préparé à cet effet comme suit: mélange de l'organopolysiloxane (i) et de l'organosiloxane (ii) ; élimination de l'air du mélange ; moulage et vulcanisation au four à 100°C pendant 30 minutes; refroidissement à température ambiante puis mesure de la 'dureté. La densité est aussi déterminée sur ce bloc d'élastomère.

En particulier, la dureté Shore peut varier de 1 à 100 et est avantageusement inférieure ou égale à 80 et mieux inférieure à 65 par exemple comprise entre 5 et 50 (bornes incluses).

Les organopolysiloxanes de la composition de l'invention sont par exemple ceux commercialisés sous les noms BY 29-122 et BY 29-119 par la société Dow-Corning Toray. On peut aussi utiliser un mélange de ces produits commerciaux.

Un bloc d'élastomère selon le produit BY-29119 présente une dureté de 30 et selon le produit BY-29122 une dureté de 7. La densité est de 0,97 à 0,98.

De façon préférentielle, la poudre d'organopolysiloxane élastomérique est présente dans la composition à un taux de 0,1 à 70 %, de préférence 4 à 70 % et mieux de 4 à 50 %, ce qui correspond à un taux de polymère en matière active de 0,5 à 65% en poids et mieux de 3 à 45%. Elle constitue, en fait, une charge hydrodispersible.

En particulier, les particules d'organopolysiloxane élastomérique (en matière active) ont une taille allant de 0,1 à 500 µm, de préférence de 3 à 200 µm et mieux de 10 et 20 µm. Ces particules peuvent être sphériques, plates ou amorphes avec, de préférence, une forme sphérique. La taille des particules du produit BY-29119 et du produit BY-29122 est de 4,5 µm.

Ces particules d'organopolysiloxane, pour se disperser de façon stable dans l'eau, peuvent être associées à un ou plusieurs tensioactifs non ioniques, cationiques ou anioniques de HLB ≥ 8. L'étape (c) est obtenue en particulier en présence d'émulsifiant non-ionique.

La proportion de tensioactifs est de préférence de 0,1 à 20 parties en poids pour 100 parties en poids d'organopolysiloxane élastomérique, et mieux, de 0,5 à 10 parties en poids (cf. description du document JP-A-10/175816).

A la dispersion de poudre d'organopolysiloxane élastomérique et de fibres peut être associée une phase grasse contenant un ou plusieurs corps gras liquides à température. ambiante, appelées huiles, tels que ceux décrits dans le document JP-A-10 /175816, une ou plusieurs cires ou une ou plusieurs gommes solides à température ambiante, un ou plusieurs corps gras pâteux, d'origine animale, végétale, minérale ou synthétique, leurs mélanges ainsi que des poudres ou charges inorganiques telles que celles décrites dans ce document.

Cette phase grasse additionnelle peut être quelconque et contenir en particulier des produits fluides à température ambiante et pression atmosphérique, appelés huiles, comme les huiles siliconées, fluorées, fluorosiliconées, hydrocarbonées éventuellement partiellement siliconées. Ces huiles peuvent être d'origine animale, végétale, minérale ou synthétique. Ces huiles peuvent être volatiles à température ambiante et pression atmosphérique. Par huile volatile, on entend en particulier une huile susceptible de s'évaporer, en moins d'une heure, au contact de la peau ou des lèvres.

Par "phase grasse", il faut comprendre un milieu non aqueux, non miscible à l'eau, contenant un ou plusieurs corps gras choisis parmi les composés ayant au moins 10 atomes de carbone et mieux 16 atomes de carbone, les composés siliconés, les composés fluorés et leurs mélanges. Ne sont pas considérés comme corps gras les solvants organiques utilisés classiquement dans les vernis à ongles.

Par "huile hydrocarbonée", on entend une huile contenant majoritairement des atomes de carbone et des atomes d'hydrogène et en particulier des chaînes alkyle ou alcényle comme les alcanes ou alcènes mais aussi une huile à chaîne alkyle ou alcényle comportant un ou des groupements éther, ester ou acide caboxylique.

Comme huiles utilisables dans la composition de l'invention, on peut citer notamment :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité;
- les esters et les éthers de synthèse notamment d'acides gras comme les huiles.de formule R¹COOR² dans laquelle R¹ représente le reste d'un acide gras supérieur comportant de 7 à 29 atomes de carbone et R² représente une chaîne hydrocarbonée ramifiée ou non contenant de 3 à 30 atomes de carbone comme par exemple l'huile de Purcellin l'isononanoate d'isononyle, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol comme le tétraiso stéarate de pentaérythrytyle ;
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras ayant de 12 à 26 atomes de carbone comme l'octyl dodécanol, le 2-butyloctanol, le 2-hexyldécanol, le 2-undécylpentadécanol ou l'alcool oléique ;
- les huiles fluorées partiellement hydrocarbonées et/ou siliconées comme celles décrites dans le document JP-A-2-295912 ;
- les huiles siliconées comme les polyméthylsiloxanes (PDMS) volatiles ou non à chaîne siliconée linéaire ou cyclique, liquides ou pâteux à température ambiante les polydiméthylsiloxanés comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényltriméthylsiloxy diphényl-siloxanes, les diphényl diméthicones, les diphénylméthyldiphényl trisiloxanes, les 2-phényléthyl triméthyl-siloxysilicates, et les polyméthylphénylsiloxanes ;
- leurs mélanges.

Ces huiles peuvent représenter de 0 à 98,80 % du poids total de la composition, de préférence de 0,5 à 80 % et mieux de 1 70 %.

Avantageusement, la composition selon l'invention peut contenir des agents structurants de la phase grasse liquide comme les cires, les gommes et les charges. Les cires peuvent être hydrocarbonées (ne contenant que des atomes de carbone et d'hydrogène), fluorées et/ou siliconées ou leurs mélanges, qui peuvent être solides ou semi-solides (sous forme d'une pâte) à température ambiante et éventuellement comporter des fonctions ester, hydroxyle ou thiol. En particulier, ces cires présentent une température de fusion supérieure à 45 °C.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyl diméthicones et des alcoxy diméthicones. Par ailleurs, ces chaînes alkyle peuvent comporter une ou plusieurs fonctions ester.

Comme autres cires utilisables dans l'invention, on peut citer les cires d'origine animale comme la lanoline, la cire d'abeille ; les cires d'origine végétale telles que la cire de Carnauba ou de Candellila ; les cirés d'origine minérale, par exemple de paraffine, de lignite ou les cires microcristallines, la cérésine ou l'ozokérite ; les cires synthétiques comme les cires de polyéthylène, les cires de Fischer-Tropsch et leurs mélanges.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un stick.

D'une manière générale, la composition peut comprendre de 0 à 50% du poids total de la composition de cire et de préférence de 5 à 30%.

La composition de l'invention peut comprendre, en outre, tout additif complémentaire usuellement utilisé dans le domaine concerné, tel que des matières colorantes comme les pigments, les nacres, les colorants hydrosolubles ou liposolubles, des antioxydants, des huiles essentielles, des conservateurs, des actifs cosmétiques ou dermatologiques comme des émollients, des hydratants (glycérine), des vitamines, des acides gras essentiels, de la lanoline liquide, et des filtres solaires lipophiles ou hydrophiles, des polymères liposolubles notamment hydrocarbonés tels que les polyalkylènes, des neutralisants, des gélifiants ou épaississants de phase grasse liquide, des parfums et leurs mélanges.

Ces additifs peuvent être présents dans la composition selon les quantités habituellement utilisées et par exemple à raison de 0 à 20% du poids total de la composition et mieux de 0,1 à 10%.

Avantageusement, la composition de l'invention contient comme additif complémentaire un ou plusieurs gélifiants ou épaississants de phase aqueuse. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de rhamsan, de karoya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisé tels que les «Carbopol» ou «Carbomer» de la Société Goodrich (Carbomer 980 par exemple neutralisé par de la triéthanolamine -TEA en abréviation-) ; les polymères poly(métha)crylates de glycéryle; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs et leurs mélanges. Ces gélifiants peuvent représenter de 0 à 60 % du poids total de la composition et notamment de 0,1 à 50 %.

Selon l'invention, le gélifiant de phase aqueuse est de préférence choisi parmi la gomme de xanthane, les argiles (bentone ou laponite), les polyuréthanes associatifs, les épaississants cellulosiques, notamment l'hydroxyéthyl cellulose, et les acides polyacryliques réticulés au moins partiellement neutralisés.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée. En particulier, ces additifs ne devront pas nuire à l'homogénéité, la stabilité, le confort, la matité, la fraîcheur et la résistance à l'eau de la composition.

La composition selon l'invention peut se présenter sous la forme d'un produit coloré et notamment de maquillage de la peau, en particulier un fond de teint, un blush, un fard à joues ou à paupières, un mascara, un eye-liner, un produit anti-cernes en stick, un vernis à ongles ou de maquillage des lèvres comme un rouge à lèvres ou un brillant à lèvres, présentant éventuellement des propriétés de soin ou de traitement, un tatouage corps. Elles peuvent également se présenter sous forme non colorée, contenant éventuellement des actifs cosmétiques ou dermatologiques. Elle peut alors être utilisée comme base de soin pour les lèvres (baumes à lèvres, protégeant les lèvres du froid et/ou du soleil et/ou du vent) ou base fixante à appliquer sur un rouge à lèvres classique.

La composition de l'invention peut également se présenter sous la forme d'une composition dermatologique ou cosmétique de traitement ou de soin de la peau (y compris le cuir chevelu), de fibres kératiniques (cheveux, cils, sourcils), des ongles ou des lèvres ou sous forme d'une composition de protection solaire ou de bronzage artificiel, ou encore sous forme d'un produit nettoyant ou démaquillant de la peau ou des fibres kératiniques, un produit déodorant ou encore un produit parfumant. Elle se présente alors sous forme non colorée, contenant éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), crème de soin de jour ou de nuit. Elle peut, en outre, se présenter sous forme de shampooing traitant ou non, colorant ou non, de produit après shampooing.

Bien entendu la composition de l'invention doit être cosmétiquement ou dermatologiquement acceptable, à savoir contenir un milieu physiologiquement acceptable non toxique et susceptible d'être appliquée sur la peau (y compris l'intérieur des paupières) ou les lèvres du visage d'êtres humains ainsi que sur les phanères d'êtres humains.

De façon préférentielle, la composition de l'invention peut comprendre une matière colorante contenant notamment une phase particulaire, généralement présente à raison de 0 à 60 % du poids total de la composition, de préférence de 5 à 35 %, et qui peut comprendre des pigments et/ou des nacres et/ou des charges habituellement utilisés dans les compositions cosmétiques. La matière colorante peut aussi consister en des colorants solubles dans le milieu et notamment lipophiles ou hydrophiles comme le rouge ou le brun soudan, le bleu de méthylène, le β-carotène, le jus de betterave ; ils peuvent représenter de 0 à 6 % du poids total de la composition.

Par pigments, il faut comprendre des particules blanches ou colorées, minérales ou organiques, insolubles dans le milieu de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 60 % du poids de la composition finale, de préférence à raison de 0,05 à 25 % et mieux de 4 à 20%. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium et leurs mélanges.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le mica, la silice, les poudres de Nylon® (Orgasol® notamment de chez Atochem) et de polyéthylène, le Téflon®, l'amidon, le nitrure de bore, des microsphères de copolymères telles que l'Expancel® (Nobel Industrie), le Polytrap® (Dow Corning) les microbilles de résine de silicone (Tospearl® de Toshiba, par exemple) et leurs mélanges.

La composition selon l'invention peut être fabriquée à froid ou par chauffage d'un ou plusieurs organopolysiloxanes élastomériques sous forme de poudre dispersée dans de l'eau, ajout d'un ou plusieurs pigments, d'une ou plusieurs charges et/ou d'un ou plusieurs autres additifs, ajout éventuel de la phase grasse à l'état liquide (notamment portée à la température de fusion des cires la plus élevée), puis émulsification si nécessaire. Elle peut aussi être obtenue par extrusion comme décrit dans la demande EP-A-667 146. Ce procédé consiste à malaxer la pâte (cires + huiles + additifs + pigments) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

L'invention est illustrée plus en détail dans les exemples suivants. Les pourcentages sont donnés en poids.

| **Exemple 1 : Fond de teint** | |
|---|---|
| ***Phase huileuse*** | |
| - Cyclopentasiloxane | 10 % |
| - KSG-21 | 20.4% |
| - Pigments | 10 % |

| ***Phase aqueuse*** | |
|---|---|
| - Trefil BY29-119 | 5 % |
| - Xanthane | 0.3 % |
| - Fibre de polyamide | 2.5 % |
| - Propylène glycol | 4 % |
| - Conservateurs | qs |
| - Eau | sp 100 |

*Préparation* : On introduit à température ambiante (25°C) les fibres dans le propylène glycol et on soumet l'ensemble à une agitation de 1000 tr/min pendant 30 min avec une turbine Rayneri. Par ailleurs, on introduit les pigments dans le mélange huile/KSG 21, sous agitation dans une turbine tricylincre. On introduit le mélange propylène glycol/fibres dans le mélange KSG 21/huile/pigments.
On obtient un fond de teint frais à l'application, doux qui se travaille facilement, assurant un maquillage homogène uniforme et esthétique.

| **Exemple 2 : Gel corps teinté** | |
|---|---|
| - Trefil BY29-119 | 32 % |
| - Carbomer 980 | 0.6 % |
| - Triéthanolamine (neutralisant) | 0.6 % |
| - Fibres de polyamide (0.3mm de long) | 1 % |
| - Propylène glycol | 1.8 % |
| - Pigments | 5 % |
| - Conservateurs | qs |
| - Eau | qsp 100 |

### Préparation :

Ce gel crème coloré est réalisé en ajoutant l'organopolysiloxane à l'eau à température ambiante, ensuite en ajoutant le gélifiant neutralisé, puis les pigments et les conservateurs. Parallèlement; on introduit les fibres à température ambiante dans le propylène glycol et on soumet le mélange obtenu à une agitation de 1000 tr/min pendant 30 min avec une turbine Rayneri. On introduit enfin le mélange fibres/propylène glycol dans le gel d'organopolysiloxane puis on mélange le tout sous agitation Rayneri pendant 10 à 15 min. Ce gel est destiné notamment au maquillage du corps ou tatouage. Il permet une application sur de petites zones du corps, avec un contour net. Le film déposé est uniforme. Le gel confère un maquillage homogène esthétique, doux, non gras, apprécié par un panel de 6 personnes
Ce gel est très matifiant, d'une grande fraîcheur à l'application, de bonne tenue dans le temps et résistant bien à l'eau, contrairement aux produits de l'art antérieur.

## Revendications

1. Composition de soin ou de maquillage des matières kératiniques, contenant une phase aqueuse, des fibres et des particules d'organopolysiloxane solide élastomérique au moins partiellement réticulé, ces fibres et ces particules étant dispersées dans une phase aqueuse, ces fibres ayant une longueur très supérieure à leur diamètre.

2. Composition selon la revendication 1, **caractérisée en ce que** l'organopolysiloxane élastomérique est obtenu par réaction d'addition et de réticulation, en présence d'un catalyseur, d'au moins :
- un organopolysiloxane (i) ayant au moins deux groupements vinyliques en position α-ω de la chaîne siliconée par molécule ; et
- d'un organosiloxane (ii) ayant au moins un atome d'hydrogène lié à un atome de silicium par molécule.

3. Composition selon l'une quelconque des revendications précédentes, **caractérisée en ce que** l'organopolysiloxane est choisi parmi les polydiméthylsiloxanes.

4. Composition selon l'une quelconque des revendications précédentes, **caractérisée en ce que** l'organopolysiloxane élastomérique est un α-ω-diméthylvinyl polydiméthylsiloxane.

5. Composition selon l'une quelconque des revendications précédentes, **caractérisée en ce que** la dispersion de particules d'organopolysiloxane est obtenue selon les étapes suivantes :
- (a) mélange de l'organopolysiloxane (i) et de l'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

6. Composition selon la revendication précédente, **caractérisée en ce que** l'étape (c) est obtenue en présence d'un émulsifiant non-ionique.

7. Composition selon l'une quelconque des revendications précédentes, **caractérisée en ce que** les particules d'organopolysiloxane élastomérique ont une taille allant de 0,1 à 500 µm et mieux de 3 à 200 µm.

8. Composition selon l'une quelconque des revendications précédentes, **caractérisée en ce que** les particules d'organopolysiloxane présentent une dureté Shore inférieure ou égale à 80 et mieux inférieure à 65.

9. Composition selon l'une quelconque des revendications précédentes, **caractérisée en ce que** l'organopolysiloxane élastomérique représente de 0.1 à 70 % du poids total de la composition et mieux de 4 à 50%.

10. Composition selon l'une des revendications précédentes, **caractérisée en ce que** les fibres sont choisies parmi les fibres de soie, de coton, de laine, de lin, des fibres de cellulose extraites notamment du bois, des légumes ou des algues, de polyamide, de rayonne, de viscose, d'acétate notamment d'acétate de rayonne, de cellulose ou de soie, de poly-p-phénylène téréphtamide, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, des fibres chirurgicales.

11. Composition selon l'une quelconque des revendications précédentes, **caractérisée en ce que** les fibres sont des fibres d'origine synthétique.

12. Composition selon l'une quelconque des revendications précédentes, **caractérisée en ce que** les fibres sont des fibres de polyamide ou de poly-p-phénylène téréphtamide.

13. Composition selon l'une quelconque des revendications précédentes, **caractérisée en ce que** les fibres ont un titre choisi dans la gamme allant de 0,15 à 30 deniers et mieux de 0,18 à 18 deniers.

14. Composition selon l'une quelconque des revendications précédentes, **caractérisée en ce que** les fibres ont une longueur L et un diamètre D tel que UD est choisi dans la gamme allant de 3,5 à 2 500, de préférence de 5 à 500 et mieux de 5 à 150.

15. Composition selon l'une quelconque des revendications précédentes, **caractérisée en ce que** les fibres ont une longueur allant de 0,1 à 5 mm, de préférence de 0,2 à 1,6 mm.

16. Composition selon l'une quelconque des revendications précédentes, **caractérisée en ce que** les fibres ont un diamètre moyen allant de 2nm à 100µm.

17. Composition selon l'une des revendications précédentes, **caractérisée en ce que** les fibres représentent de 0,1 à 20 du poids total de la composition et mieux de 0,5 à 10.

18. Composition selon l'une quelconque des revendications précédentes, **caractérisée en ce qu'**elle contient, en outre, une phase grasse.

19. Composition selon la revendication précédente, **caractérisée en ce que** la phase grasse contient au moins un corps gras choisi parmi les huiles liquides à températures ambiantes volatiles ou non, les cires, les gommes et les corps gras pâteux, d'origine animale, végétale, minérale ou synthétique, et leurs mélanges.

20. Composition selon l'une des revendications précédentes, **caractérisée en ce qu'**elle contient au moins une huile choisie parmi le perhydrosqualène ; les triglycérides des acides heptanoïque ou octanoïque ou encore les huiles de tournesol, de maïs, de soja, de courge, de pépins de raisin, de sésame, de noisette, d'abricot, de macadamia, d'arara, de ricin, d'avocat, les triglycérides des acides caprylique/caprique, l'huile de jojoba, de beurre de karité ; les huiles de paraffine et leurs dérivés, la vaseline, les polydécènes, le polyisobutène hydrogéné tel que le parléam ; l'huile de Purcellin, le myristate d'isopropyle, l'isononanoate d'isononyle, le palmitate d'éthyl-2hexyle, le stéarate d'octyl-2-dodécyle, l'érucate d'octyl-2-dodécyle, l'isostéarate d'isostéaryle ; l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; le dioctanoate de propylène glycol, le diheptanoate de néopentylglycol, le diisononanoate de diéthylèneglycol ; les esters du pentaérythritol ; l'octyldodécanol, le 2-butyloctanol, le 2-hexyldécanol, le 2-undécylpentadécanol, l'alcool oléique ; les polyméthylsiloxanes (PDMS) volatiles ou non, linéaires ou cycliques, liquides à température ambiante ; les phényl triméthicones, les phényltriméthylsiloxy-diphényl-siloxanes, les diphényl diméthicones, les diphénylméthyldiphényl trisiloxanes, les 2-phényl-éthyl triméthylsiloxysilicates et leurs mélanges.

21. Composition selon l'une des revendications précédentes, **caractérisée en ce qu'**elle comprend, en outre, un gélifiant de phase aqueuse.

22. Composition selon la revendication précédente, **caractérisée en ce que** le gélifiant de phase aqueuse est choisi parmi la gomme de xanthane, les argiles, les polyuréthanes associatifs, les épaississants cellulosiques et les acides polyacryliques réticulés au moins partiellement neutralisés.

23. Composition selon l'une des revendications précédentes **caractérisée en ce qu'**elle comprend, entre autre, une matière colorante.

24. Composition selon l'une des revendications précédentes, **caractérisée en ce qu'**elle comprend, en outre, une phase particulaire présente à raison de 0 à 60 % du poids total de la composition, de préférence 5 à 35 %.

25. Composition selon, l'une des revendications précédentes, **caractérisée en ce qu'**elle contient, en outre, au moins un actif cosmétique ou dermatologique.

26. Composition selon l'une des revendications précédentes, **caractérisée en ce qu'**elle se présente sous forme d'un gel hydrophile, d'émulsion huile-dans-eau ou eau-dans-huile, rigide ou souple, éventuellement coulé en stick ou en coupelle.

27. Composition selon l'une des revendications précédentes, **caractérisée en ce qu'**elle contient, en outre, au moins un ingrédient choisi parmi les antioxydants, les huiles essentielles, les conservateurs, les parfums, les polymères liposolubles, les gélifiants de phase grasse liquide, les cires, les gommes, les charges, les dispersants, les composés miscibles à l'eau et leurs mélanges.

28. Composition selon l'une des revendications précédentes, **caractérisée en ce qu'**elle se présente sous forme d'une composition de fond de teint, de fard à joues ou à paupières, d'un produit anti-cernes, d'un maquillage du corps, d'un rouge à lèvres, d'un eye-liner, d'un mascara, d'un vernis à ongles, d'une base de soin ou d'une base fixante pour les lèvres, d'un produit dermatologique ou de soin de la peau ou des fibres kératiniques, d'une composition de protection solaire ou de bronzage artificiel, d'un produit nettoyant de la peau ou des fibres kératiniques.

29. Procédé cosmétique de soin ou de traitement des matières kératiniques des êtres humains, comprenant l'application sur ces matières kératiniques de la composition cosmétique selon l'une quelconque des revendications précédentes.

30. Procédé cosmétique pour obtenir un maquillage homogène, consistant à introduire dans une composition cosmétique des particules d'un organopolysiloxane solide élastomérique au moins partiellement réticulé en dispersion dans une phase aqueuse, conforme à l'une des revendications 2 à 9 et des fibres conformes à l'une des revendications 10 à 17.

31. Utilisation cosmétique de particules d'organopolysiloxane solide élastomérique au moins partiellement réticulé et de fibres, les particules et les fibres étant dispersées dans une phase aqueuse, et les fibres ayant une longueur très supérieure à leur diamètre, dans une composition de soin de maquillage de matières kératiniques, pour conférer à ladite composition des propriétés de résistance à l'eau et/ou conférer un maquillage ou un soin homogène et/ou matifiant et/ou frais.

32. Utilisation selon la revendication 31, **caractérisée en ce que** les particules d'organopolysiloxanes sont conformes à l'une des revendications 2 à 9.

33. Utilisation selon la revendication 31 ou 32, **caractérisée en ce que** les fibres sont conformes à l'une des revendications 10 à 17.

## Patentansprüche

1. Zusammensetzung zur Pflege oder zum Schminken von Keratinsubstanzen, die eine wäßrige Phase, Fasern und Partikel eines zumindest teilweise vernetzten, elastomeren, festen Organopolysiloxans enthält, wobei die Fasern und die Partikel in einer wäßrigen Phase dispergiert sind und wobei die Fasern eine Länge aufweisen, die deutlich über ihrem Durchmesser liegt.

2. Zusammensetzung nach Anspruch 1, **dadurch gekennzeichnet, daß** das elastomere Organopolysiloxan in Gegenwart eines Katalysators durch Addition und Vernetzung von mindestens:
- einem Organopolysiloxan (i), das pro Molekül in α-ω-Stellung der Siliconkette mindestens zwei Vinylgruppen aufweist; und
- einem Organosiloxan (ii), das pro Molekül mindestens ein Wasserstoffatom, das an ein Siliciumatom gebunden ist, aufweist, hergestellt wird.

3. Zusammensetzung nach einem der vorhergehenden Ansprüche, **dadurch gekennzeichnet, daß** das Organopolysiloxan unter den Polydimethylsiloxanen ausgewählt ist.

4. Zusammensetzung nach einem der vorhergehenden Ansprüche, **dadurch gekennzeichnet, daß** das elastomere Organopolysiloxan ein α-ω-Dimethylvinyl-polydimethylsiloxan ist.

5. Zusammensetzung nach einem der vorhergehenden Ansprüche, **dadurch gekennzeichnet, daß** die Dispersion von Partikeln des Organopolysiloxans gemäß den folgenden Schritten hergestellt wird:
(a) Mischen des Organopolysiloxans (i) und des Organosiloxans (ii);
(b) Zugabe der wäßrigen Phase, die einen Emulgator enthält, zu dem Gemisch des Schrittes (a);
(c) Emulgieren der wäßrigen Phase und des Gemisches;
(d) Zugabe von warmem Wasser zu der Emulsion der Phase (c); und
(e) Polymerisation des Organopolysiloxans (i) und des Organosiloxans (ii) in Emulsion in Gegenwart eines Platinkatalysators.

6. Zusammensetzung nach dem vorhergehenden Anspruch, **dadurch gekennzeichnet, daß** Schritt (c) in Gegenwart eines nichtionischen Emulgators durchgeführt wird.

7. Zusammensetzung nach einem der vorhergehenden Ansprüche, **dadurch gekennzeichnet, daß** die Partikel des elastomeren Organopolysiloxans eine Größe im Bereich von 0,1 bis 500 µm und noch besser 3 bis 200 µm aufweisen.

8. Zusammensetzung nach einem der vorhergehenden Ansprüche, **dadurch gekennzeichnet, daß** die Organopolysiloxan-Partikel eine Shore-Härte von höchstens 80 und noch besser unter 65 aufweisen.

9. Zusammensetzung nach einem der vorhergehenden Ansprüche, **dadurch gekennzeichnet, daß** das elastomere Organopolysiloxan 0,1 bis 70 % und noch besser 4 bis 50 % des Gesamtgewichts der Zusammensetzung ausmacht.

10. Zusammensetzung nach einem der vorhergehenden Ansprüche, **dadurch gekennzeichnet, daß** die Fasern unter den Fasern aus Seide, Baumwolle, Wolle, Leinen, Cellulose, die insbesondere aus Holz, Gemüse oder Algen gewonnen wurden, Polyamid, Reyon, Viscose, Acetat und insbesondere dem Acetat von Reyon, Cellulose oder Seide, Poly-p-phenylenterephthamid, Acrylfasern, insbesondere aus Polymethylmethacrylat oder Poly-2-hydroxyethylmethacrylat, aus Polyolefin und insbesondere Polyethylen oder Polypropylen, Glas, Siliciumdioxid, Aramid, Kohlenstoff und insbesondere Kohlenstoff in Form von Graphit, Teflon® , unlöslichem Kollagen, Polyestern, Polyvinylchlorid oder Polyvinylidenchlorid, Polyvinylalkohol, Polyacrylnitril, Chitosan, Polyurethan, Polyethylenphthalat, Fasern aus Polymergemischen und chirurgischen Fasern ausgewählt sind.

11. Zusammensetzung nach einem der vorhergehenden Ansprüche, **dadurch gekennzeichnet, daß** die Fasern synthetischer Herkunft sind.

12. Zusammensetzung nach einem der vorhergehenden Ansprüche, **dadurch gekennzeichnet, daß** die Fasern Polyamidfasern oder Fasern aus Poly-p-phenylenterephthamid sind.

13. Zusammensetzung nach einem der vorhergehenden Ansprüche, **dadurch gekennzeichnet, daß** die Fasern eine Feinheit aufweisen, die im Bereich von 0,15 bis 30 Denier und besser 0,18 bis 18 Denier ausgewählt ist.

14. Zusammensetzung nach einem der vorhergehenden Ansprüche, **dadurch gekennzeichnet, daß** die Fasern eine Länge und einen Durchmesser haben, die so gewählt sind, daß L/D im Bereich von 3,5 bis 2500, vorzugsweise 5 bis 500 und besser 5 bis 150 liegt.

15. Zusammensetzung nach einem der vorhergehenden Ansprüche, **dadurch gekennzeichnet, daß** die Fasern eine Länge von 0,1 bis 5 mm und vorzugsweise 0,2 bis 1,6 mm aufweisen.

16. Zusammensetzung nach einem der vorhergehenden Ansprüche, **dadurch gekennzeichnet, daß** die Fasern einen mittleren Durchmesser von 2 nm bis 100 µm aufweisen.

17. Zusammensetzung nach einem der vorhergehenden Ansprüche, **dadurch gekennzeichnet, daß** die Fasern 0,1 bis 20 des Gesamtgewichts der Zusammensetzung und besser 0,5 bis 10 ausmachen.

18. Zusammensetzung nach einem der vorhergehenden Ansprüche, **dadurch gekennzeichnet, daß** sie ferner eine Fettphase enthält.

19. Zusammensetzung nach dem vorhergehenden Anspruch, **dadurch gekennzeichnet, daß** die Fettphase mindestens eine Fettsubstanz enthält, die unter den bei Raumtemperatur flüssigen, flüchtigen oder nicht flüchtigen Ölen, Wachsen, Gummis und pastösen Fettsubstanzen tierischer, pflanzlicher, mineralischer oder synthetischer Herkunft oder deren Gemischen ausgewählt ist.

20. Zusammensetzung nach einem der vorhergehenden Ansprüche, **dadurch gekennzeichnet, daß** sie mindestens ein Öl enthält, das ausgewählt ist unter; Perhydrosqualen; Triglyceriden von Heptansäure oder Octansäure, Sonnenblumenöl, Maisöl, Sojaöl, Kürbiskemöl, Traubenkernöl, Sesamöl, Haselnußöl, Aprikosenöl, Macadamiaöl, Araraöl, Ricinusöl, Avocadoöl, Triglyceriden von Capryl/Caprinsäure, Jojobaöl, Sheabutter; Paraffinölen und deren Derivaten, Vaseline, Polydecenen und hydriertem Polyisobuten, beispielsweise Parleam; Purcellinöl, Isopropylmyristat, Isononylisononanoat, 2-Ethylhexylpalmitat, 2-Octyldodecylstearat, 2-Octyldodecylerucat, Isostearylisostearat; Isostearyllactat, Octylhydroxystearat, Octyldodecylhydroxystearat, Diisostearylmalat, Triisocetylcitrat, Heptanoaten, Octanoaten oder Decanoaten von Fettalkoholen; Propylenglykoldioctanoat, Neopentylglykoldiheptanoat, Diethylenglykoldiisononanoat; Estern von Pentaerythrit; Octyldodecanol, 2-Butyloctanol, 2-Hexyldecanol, 2-Undecylpentadecanol oder Oleylalkohol; geradkettigen oder cyclischen, bei Raumtemperatur flüssigen, flüchtigen oder nicht flüchtigen Polymethylsiloxanen (PDMS); Phenyltrimethiconen, Phenyitrimethylsiloxydipherlylsiloxanen, Diphenyldimethiconen, Diphenylmethyldiphenyltrisiloxanen, 2-Phenylethyltrimethylsiloxysilicaten und deren Gemischen.

21. Zusammensetzung nach einem der vorhergehenden Ansprüche, **dadurch gekennzeichnet, daß** sie ferner mindestens einen Gelbildner für die wäßrige Phase enthält.

22. Zusammensetzung nach dem vorhergehenden Anspruch, **dadurch gekennzeichnet, daß** der Gelbildner für die wäßrige Phase unter Xanthangummi, Tonen, assoziativen Polyurethanen, Verdickungsmitteln auf Cellulosebasis und vernetzten, zumindest teilweise neutralisierten Polyacrylsäuren ausgewählt ist.

23. Zusammensetzung nach einem der vorhergehenden Ansprüche, **dadurch gekennzeichnet, daß** sie ferner ein Farbmittel enthält.

24. Zusammensetzung nach einem der vorhergehenden Ansprüche, **dadurch gekennzeichnet, daß** sie ferner eine Partikelphase enthält, die in einem Mengenanteil von 0 bis 60 % des Gesamtgewichts der Zusammensetzung und vorzugsweise im Bereich von 5 bis 35 % vorliegt.

25. Zusammensetzung nach einem der vorhergehenden Ansprüche, **dadurch gekennzeichnet, daß** die Zusammensetzung ferner mindestens einen kosmetischen oder dermatologischen Wirkstoff enthält.

26. Zusammensetzung nach einem der vorhergehenden Ansprüche, **dadurch gekennzeichnet, daß** sie in Form eines hydrophilen Gels oder einer starren oder weichen Öl-in-Wasser-Emulsion oder Wasser-in-Öl-Emulsion vorliegt, die gegebenenfalls in Stiftform vorliegen oder in ein Tiegelchen gegossen wurden.

27. Zusammensetzung nach einem der vorhergehenden Ansprüche, **dadurch gekennzeichnet, daß** sie außerdem mindestens einen Bestandteil enthält, der unter den Antioxidantien, etherischen Ölen, Konservierungsmitteln, Parfums, fettlöslichen Polymeren, Gelbildnern für die flüssige Fettphase, Wachsen, Gummen/Gummis, Füllstoffen, Dispergiermitteln, mit Wasser mischbaren Verbindungen und deren Gemischen ausgewählt ist.

28. Zusammensetzung nach einem der vorhergehenden Ansprüche, **dadurch gekennzeichnet, daß** sie als Make-up, Wangenrouge, Lidschatten, Produkt gegen Augenringe, Produkt zum Schminken des Körpers, Lippenstift, Eyeliner, Mascara, Nagellack, Pflegebasis für die Lippen oder fixierende Masse für die Lippen, dermatologisches Produkt oder Produkt zur Pflege der Haut oder der Keratinsubstanzen, Zusammensetzung zum Sonnenschutz oder zur Selbstbräunung oder als Produkt zur Reinigung der Haut oder der Keratinfasern vorliegt.

29. Kosmetisches Verfahren zur Pflege oder zur Behandlung von Keratinsubstanzen des Menschen, das umfaßt, eine kosmetische Zusammensetzung nach einem der vorhergehenden Ansprüche auf die Keratinsubstanzen aufzutragen.

30. Kosmetisches Verfahren zur Erzeugung einer homogenen Schminke, das darin besteht, Partikel eines zumindest zum Teil vernetzten, festen, elastomeren Organopolysiloxans, die in einer wäßrigen Phase dispergiert sind, nach einem der Ansprüche 2 bis 9 und Fasern nach einem der Ansprüche 10 bis 17 in eine kosmetische Zusammensetzung einzuarbeiten.

31. Kosmetische Verwendung von Partikeln eines zumindest zum Teil vernetzten, festen, elastomeren Organopolysiloxans, die in einer wäßrigen Phase dispergiert sind, wobei die Fasern eine Länge aufweisen, die deutlich über ihrem Durchmesser liegt, in einer kosmetischen Zusammensetzung zur Pflege oder zum Schminken von Keratinsubstanzen, um die Zusammensetzung wasserfest zu machen und/oder eine Schminke oder ein Pflegeprodukt zu erzeugen, das homogen und/oder frisch ist und/oder mattierend wirkt.

32. Verwendung nach Anspruch 31, **dadurch gekennzeichnet, daß** es sich um Partikel von Organopolysiloxanen nach einem der Ansprüche 2 bis 9 handelt.

33. Verwendung nach Anspruch 31 oder 32, **dadurch gekennzeichnet, daß** es sich um Fasern nach einem der Ansprüche 10 bis 17 handelt.

## Claims

1. Care or make-up composition for keratin substances, containing an aqueous phase, fibres and particles of an at least partially crosslinked solid elastomeric polyorganosiloxane, these fibres and these particles being dispersed in an aqueous phase, these fibres having a length which is very much greater than their diameter.

2. Composition according to Claim 1, **characterized in that** the elastomeric polyorganosiloxane is obtained by addition reaction and crosslinking, in the presence of a catalyst, of at least:
- one polyorganosiloxane (i) containing at least two vinyl groups in α-ω position of the silicone chain per molecule; and
- one organosiloxane (ii) containing at least one hydrogen atom linked to a silicon atom per molecule.

3. Composition according to either of the preceding claims, **characterized in that** the polyorganosiloxane is chosen from polydimethylsiloxanes.

4. Composition according to any one of the preceding claims, **characterized in that** the elastomeric polyorganosiloxane is an α,ω-dimethylvinyl polydimethylsiloxane.

5. Composition according to any one of the preceding claims, **characterized in that** the dispersion of polyorganosiloxane particles is obtained according to the following steps:
- (a) mixing the polyorganosiloxane (i) and the organosiloxane (ii);
- (b) adding the aqueous phase containing an emulsifier to the mixture from step (a);
- (c) emulsifying the aqueous phase and the said mixture;
- (d) adding hot water to the emulsion from step (c) ; and
- (e) polymerizing the polyorganosiloxane (i) and the organosiloxane (ii) in emulsion in the presence of a platinum catalyst.

6. Composition according to the preceding claim, **characterized in that** step (c) is obtained in the presence of a nonionic emulsifier.

7. Composition according to any one of the preceding claims, **characterized in that** the particles of elastomeric polyorganosiloxane have a size ranging from 0.1 µm to 500 µm and better still from 3 µm to 200 µm.

8. Composition according to any one of the preceding claims, **characterized in that** the polyorganosiloxane particles have a Shore hardness of less than or equal to 80 and better still less than 65.

9. Composition according to any one of the preceding claims, **characterized in that** the elastomeric polyorganosiloxane represents from 0.1% to 70% relative to the total weight of the composition, and better still from 4% to 50%.

10. Composition according to one of the preceding claims, **characterized in that** the fibres are chosen from silk fibre, cotton fibre, wool fibre, flax fibre, cellulose fibres extracted in particular from wood, from plants or from algae, polyamide fibre, rayon fibre, viscose fibre, acetate fibre, in particular silk, cellulose or rayon acetate fibre, poly(p-phenyleneterephthamide) fibre, acrylic fibre, in particular polymethyl methacrylate fibre or poly(2-hydroxyethyl methacrylate) fibre, polyolefin fibre and in particular polyethylene or polypropylene fibre, glass fibre, silica fibre, aramide fibre, carbon fibre, in particular in graphite form, Teflon® fibre, insoluble collagen fibre, polyester fibre, polyvinyl chloride fibre or polyvinylidene chloride fibre, polyvinyl alcohol fibre, polyacrylonitrile fibre, chitosan fibre, polyurethane fibre, polyethylene phthalate fibre, fibres from mixtures of polymers, and surgical fibres.

11. Composition according to any one of the preceding claims, **characterized in that** the fibres are fibres of synthetic origin.

12. Composition according to any one of the preceding claims, **characterized in that** the fibres are polyamide fibres or poly(p-phenyleneterephthamide) fibres.

13. Composition according to any one of the preceding claims, **characterized in that** the fibres have a yarn count which is chosen in the range from 0.15 denier to 30 denier and better still from 0.18 denier to 18 denier.

14. Composition according to any one of the preceding claims, **characterized in that** the fibres have a length L and a diameter D such that L/D is chosen within the range from 3.5 to 2500, preferably from 5 to 500 and better still from 5 to 150.

15. Composition according to any one of the preceding claims, **characterized in that** the fibres have a length ranging from 0.1 mm to 5 mm, preferably from 0.2 mm to 1.6 mm.

16. Composition according to any one of the preceding claims, **characterized in that** the fibres have an average diameter ranging from 2 nm to 100 µm.

17. Composition according to one of the preceding claims, **characterized in that** the fibres represent from 0.1% to 20% relative to the total weight of the composition, and better still from 0.5% to 10%.

18. Composition according to any one of the preceding claims, **characterized in that** it also contains a fatty phase.

19. Composition according to the preceding claim, **characterized in that** the fatty phase contains at least one fatty substance chosen from volatile or non-volatile oils that are liquid at room temperature, waxes, gums and pasty fatty substances of animal, plant, mineral or synthetic origin, and mixtures thereof.

20. Composition according to one of the preceding claims, **characterized in that** it contains at least one oil chosen from perhydrosqualene; heptanoic or octanoic acid triglycerides or alternatively sunflower oil, corn oil, soybean oil, marrow oil, grapeseed oil, sesame oil, hazelnut oil, apricot oil, macadamia oil, arara oil, castor oil, avocado oil, caprylic/capric acid triglycerides, jojoba oil or Karite butter; liquid paraffins and derivatives thereof, petroleum jelly, polydecenes or hydrogenated polyisobutene such as parleam; purcellin oil, isopropyl myristate, isononyl isononanoate, 2-ethylhexyl palmitate, 2-octyldodecyl stearate, 2-octyldodecyl erucate or isostearyl isostearate; isostearyl lactate, octyl hydroxystearate, octyldodecyl hydroxystearate, diisostearyl malate, triisocetyl citrate or fatty alkyl heptanoates, octanoates or decanoates; propylene glycol dioctanoate, neopentyl glycol diheptanoate or diethylene glycol diisononanoate; pentaerythritol esters; octyldodecanol, 2-butyloctanol, 2-hexyldecanol, 2-undecylpentadecanol or oleyl alcohol; volatile or non-volatile, linear or cyclic polydimethylsiloxanes (PDMSs) that are liquid at room temperature; phenyl trimethicones, phenyltrimethylsiloxydiphenylsiloxanes, diphenyl dimethicones, diphenylmethyldiphenyltrisiloxanes and 2-phenylethyl trimethylsiloxysilicates, and mixtures thereof.

21. Composition according to one of the preceding claims, **characterized in that** it also comprises an aqueous-phase gelling agent.

22. Composition according to the preceding claim, **characterized in that** the aqueous-phase gelling agent is chosen from xanthan gum, clays, associative polyurethanes, cellulosic thickeners and crosslinked, at least partially neutralized polyacrylic acids.

23. Composition according to one of the preceding claims, **characterized in that** it also comprises a dyestuff.

24. Composition according to one of the preceding claims, **characterized in that** it also comprises a particulate phase which is present in a proportion of from 0% to 60% relative to the total weight of the composition, preferably 5% to 35%.

25. Composition according to one of the preceding claims, **characterized in that** it also contains at least one cosmetic or dermatological active agent.

26. Composition according to one of the preceding claims, **characterized in that** it is in the form of a hydrophilic gel or a rigid or soft oil-in-water or water-in-oil emulsion, optionally cast as a stick or a dish.

27. Composition according to one of the preceding claims, **characterized in that** it also contains at least one ingredient chosen from antioxidants, essential oils, preserving agents, fragrances, liposoluble polymers, liquid-fatty-phase gelling agents, waxes, gums, fillers, dispersants and water-miscible compounds, and mixtures thereof.

28. Composition according to one of the preceding claims, **characterized in that** it is in the form of a foundation, face powder or eyeshadow composition, a concealer product, a make-up product for the body, a lipstick, an eyeliner, a mascara, a nail varnish, a care base or a fixing base for the lips, a dermatological product or a care product for the skin or keratin fibres, an antisun composition or an artificial tanning composition, or a cleansing product for the skin or keratin fibres.

29. Cosmetic care or treatment process for keratin substances of human beings, comprising the application to these keratin substances of the cosmetic composition according to any one of the preceding claims.

30. Cosmetic process for obtaining a homogeneous make-up, which consists in introducing into a cosmetic composition particles of an at least partially crosslinked, solid elastomeric polyorganosiloxane dispersed in an aqueous phase, in accordance with one of Claims 2 to 9, and fibres in accordance with one of Claims 10 to 17.

31. Cosmetic use of particles of at least partially crosslinked solid elastomeric polyorganosiloxane and of fibres, the particles and the fibres being dispersed in an aqueous phase, and the fibres having a length much greater than their diameter, in a care or make-up composition for keratin substances, in order to give the said composition water-resistant properties and/or to give a homogeneous and/or matt and/or fresh make-up or care effect.

32. Use according to Claim 31, **characterized in that** the polyorganosiloxane particles are in accordance with one of Claims 2 to 9.

33. Use according to Claim 31 or 32, **characterized in that** the fibres are in accordance with one of Claims 10 to 17.
